# EUROPÄISCHE PATENTSCHRIFT

(11) **EP 4 444 848 B1**
(45) Veröffentlichungstag und Bekanntmachung des Hinweises auf die Patenterteilung: **09.12.2026**
(21) Anmeldenummer: 22817275.5
(22) Anmeldetag: 15.11.2022
(51) Int. Cl.: C12M 1/32, C12M 1/00, C12M 1/12

(54) **PROBENVORRICHTUNG FÜR ZELLKULTUREN**
SAMPLING APPARATUS FOR CELL CULTURES
APPAREIL D'ÉCHANTILLONNAGE POUR CULTURES CELLULAIRES

(30) Priorität: 09.12.2021 DE 102021132560
(43) Veröffentlichungstag der Anmeldung: 16.10.2024
(73) Patentinhaber: Sabeu GmbH & Co. KG, 37154 Northeim (DE)
(72) Erfinder: BENKMANN, Dennis, 24568 Kaltenkirechen (DE)
(74) Vertreter: Deters, Frank
(86) Internationale Anmeldenummer: PCT/EP2022/081991
(87) Internationale Veröffentlichungsnummer: WO 2023/104449

(56) Entgegenhaltungen:
- EP-A1- 2 233 560
- WO-A1-2018/220190
- WO-A2-2006/131123
- DE-A1- 10 240 787
- DE-B4- 102012 100 583
- DE-T2- 69 109 651
- DE-T2- 69 635 398
- DE-U1- 202017 003 978
- FR-A1- 2 881 434

## Beschreibung

Die Erfindung betrifft eine Probenvorrichtung für Zellkulturen mit einer Multiwell-Platte, die eine Mehrzahl fest mit der Multiwell-Platte verbundener Einzelwells aufweist.

Eine derartige Probenvorrichtung ist aus der DE 10 2012 100 583 B4 bekannt. Die Einzelwells weisen dabei einen gegenüber den weiteren Einzelwells abgegrenzten Probenraum für eine Nährlösung auf, in den jeweils ein Probeneinsatz eingehängt wird.

EP 2 233 560 offenbart eine Multiwell-Platte als Probenvorrichtung mit Einzelwells und Zellkultureinsätzen, die in die Vertiefungen (46) mittels Tragarm, vor Verrutschen gesichert, in die Öffnungskontur eingehängt werden.

Der Erfindung liegt die Aufgabe zugrunde, eine Probenvorrichtung der eingangs genannten Art bereitzustellen die konstruktiv bedingt wenig fehleranfällig in der Anwendung ist.

Diese Aufgabe wird erfindungsgemäß durch eine Probenvorrichtung mit den Merkmalen des Patentanspruchs 1 gelöst. Weitere vorteilhafte Ausgestaltungen sind den Unteransprüchen 2 bis 10 zu entnehmen.

Die erfindungsgemäße Probenvorrichtung mit einem an die Form der Multiwell-Platte (4) angepassten Deckel (28) mit den Merkmalen des unabhängigen Anspruchs 1 umfasst eine Multiwell-Platte, die eine Mehrzahl fest mit der Multiwell-Platte verbundener Einzelwells aufweist. Jedes Einzelwell weist dabei einen gegenüber den weiteren Einzelwells abgegrenzten Probenraum für eine Nährlösung auf, der umseitig von einer Wandung und unten von einem sich an die Wandung anschließenden Boden begrenzt ist. Der Probenraum ist über eine dem Boden gegenüberliegende Öffnungen zugänglich, die von einer die Wandung begrenzenden Öffnungskontur umfasst ist. Jede Öffnungskontur der Wandung eines Einzelwells weist diesem zugeordnete Vertiefungen zum Einlegen von Tragarmen eines Probeneinsatzes auf. Durch die Vertiefungen wird erreicht, dass ein in ein Einzelwell in einer bestimmten Positionierung eingesetzter Probeneinsatz nicht nachträglich in eine hiervon abweichende neue Position verrutschen kann. Die in den Vertiefungen eingelegten Tragarme und darüber der Probeneinsatz werden durch die Vertiefungen an einer nachträglichen Lageveränderung gehindert. Die Unveränderlichkeit der Lage des Probeneinsatzes ist z.B. bei TEER-Messungen an Zellkulturen von großer Bedeutung. Zudem wird durch die erfindungsgemäße Ausgestaltung sichergestellt, dass nicht an den Tragarmen eines Probeneinsatzes befindliche Kontaminationen zusätzlich in ein benachbartes Einzelwell eingetragen werden, wenn der Probeneinsatz in ein Einzelwell eingesetzt wird. Auch wird durch die erfindungsgemäße Ausgestaltung verhindert, dass ein Probeneinsatz mit einem Tragarm fälschlicherweise in eine Vertiefung eines Einzelwells eingesetzt wird, die nicht diesem sondern einem benachbarten Einzelwell zugeordnet ist. Der Probeneinsatz wäre anderenfalls schief eingehängt und Messungen würden verfälscht.

In einer vorteilhaften Ausgestaltung der Erfindung weist jede der Wandungen einen gegenüber dem an das jeweilige Einzelwell unmittelbar angrenzenden Umfassungsbereich erhöhten Sicherheitsrand auf. Unter dem Umfassungsbereich wird dabei der Materialanteil der Multiwell-Platte verstanden, der unmittelbar an die Wandung des Einzelwells angrenzt. Dies kann ein flächiger Beschriftungsbereich neben den Einzelwells sein oder auch eine Bodenplatte der Multiwell-Platte. Durch diese Ausgestaltung wird erreicht, dass versehentlich neben ein Einzelwell gelangtes Probenmaterial oder auch Kontaminationen nicht in den Probenraum des Einzelwells eindringen. Zudem wird hierdurch erreicht, dass in den Vertiefungen der Einzelwells positionierte Tragarme eines Probeneinsatzes einen Sicherheitsabstand zum Umfassungsbereich nicht unterschreiten. Auf dem Umfassungsbereich versehentlich befindliche Kontaminationen können so auch nicht an die Tragarme gelangen. Mit Vorteil weist der Sicherheitsrand eine dem Probenraum zugewandte Innenseite und eine dieser gegenüberliegende Außenseite auf, wobei die Außenseite weiter in Richtung einer Bodenebene reicht, in der die Böden der Einzelwells positioniert sind, als der umlaufend an die Außenseite unmittelbar angrenzende Umfassungsbereich der Multiwell-Platte.

In einer besonders bevorzugten Ausgestaltung der Erfindung ist der Sicherheitsrand jedes Einzelwells um wenigstens 0,5 mm, insbesondere um wenigstens 1 mm gegenüber dem an das jeweilige Einzelwell unmittelbar angrenzenden Umfassungsbereich erhöht. Außerhalb der Vertiefungen ist der Sicherheitsrand hierbei höher gezogen als im Bereich der Vertiefungen. Die vorgenannte Mindesthöhe des Sicherheitsrands wird dabei aber auch im Bereich der Vertiefungen nicht unterschritten. Die vorgenannten Vorteile des Sicherheitsrands, Kontaminationen entgegenzuwirken, bleiben dadurch auch bei einer Reduzierung der Höhe des Sicherheitsrands im Bereich der Vertiefungen aufrechterhalten.

Erfindungsgemäß umfasst die Probenvorrichtung einen an die Form der Multiwell-Platte angepassten Deckel und Probeneinsätze mit einem hohlzylinderförmigen, einseitig eine obere Gehäuseöffnung aufweisenden Gehäuse, einem der Gehäuseöffnung gegenüberliegenden, membranartigen Gehäuseboden, sowie am Gehäuse angeordneten, nach außen abstehenden, zueinander beabstandeten Tragarmen. Der Deckel weist dabei innenseitig Niederhalter auf, die im geschlossenen Zustand der Probenvorrichtung mit auf der Multiwell-Platte aufgesetztem Deckel oberhalb jedes Tragarmes positioniert sind. Die Niederhalter sind dabei bevorzugt als Vorsprünge an der Innenseite des Deckels ausgebildet. Durch die Niederhalter wird ein Aufschwimmen der Probeneinsätze auf einer im Probenraum befindlichen Nährflüssigkeit verhindert. Der Probeneinsatz wird dabei durch die Niederhalter in die Nährflüssigkeit heruntergedrückt. Hierdurch wird zum einen ein ausreichender Kontakt des Bodens des Probeneinsatzes mit der Nährflüssigkeit sichergestellt und zum anderen wird verhindert, dass die Tragarme infolge des Aufschwimmens die Vertiefungen verlassen und damit die Funktion der Vertiefungen umgangen wird. Zudem wird der Gasaustausch zwischen dem Probenraum und der Umgebungsluft außerhalb der Probenvorrichtung verbessert.

Mit Vorteil weisen die Tragarme auf der dem Gehäuseboden abgewandten Oberfläche zumindest einen Vorsprung auf und die Niederhalter sind jeweils oberhalb dieser Vorsprünge positioniert. Die Vorsprünge haben dabei bevorzugt eine kugelförmig abgerundete Form. Die Kontaktfläche zwischen Niederhalter und Vorsprung, als Teil des Tragarmes, ist dadurch minimiert.

Mit Vorteil beträgt der Abstand zwischen den Niederhaltern und den Tragarmen im geschlossenen Zustand der Probenvorrichtung mit auf der Multiwell-Platte aufgesetztem Deckel nicht mehr als 0,3 mm. Das unerwünschte Aufschwimmen kann dadurch auf ein Minimum reduziert werden.

In einer besonders bevorzugten Ausgestaltung der Erfindung liegen die Niederhalter im geschlossenen Zustand der Probenvorrichtung mit auf der Multiwell-Platte aufgesetztem Deckel auf den Tragarmen auf. Das unerwünschte Aufschwimmen des Probeneinsatzes auf dem Nährmedium kann hierdurch gänzlich unterbunden werden.

In einer vorteilhaften Ausgestaltung der Erfindung hat jede der Vertiefungen in der Öffnungskontur im oberen Bereich einen Einlegebereich, der zwei sich gegenüberliegende Schenkel aufweist, die konisch zueinander verlaufen und jeweils einen Winkel zu einer Senkrechten zur Bodenebene, in der die Böden der Einzelwells liegen, von 15° bis 45°, insbesondere von 25° bis 35° einschließen. Im Einlegebereich verjüngt sich die Vertiefung nach unten hin. Durch diese Verjüngung ist ein einfacheres Einsetzen des Probeneinsatzes in die Multiwell-Platte gegeben. Die Möglichkeiten eines fehlerhaften Einsetzens sind reduziert. Zudem nimmt der Probeneinsatz infolge der Verjährung eine eng eingegrenzte Endposition ein. Der Einlegebereich erstreckt sich bevorzugt zumindest über die Hälfte der Höhe der Vertiefung.

Mit Vorteil weist die Öffnungskontur im unteren Bereich der Vertiefungen jeweils einen Fixierungsbereich auf, in dem die Öffnungskontur beiderseits jeweils senkrecht zur Bodenebene verläuft. Durch den senkrechten Verlauf der Vertiefungen im Fixierungsbereich ist es für die Tragarme schwerer möglich, die Vertiefungen ungewollt zu verlassen. Gleichzeitig bleiben aber die Vorteile der Verjüngung der Vertiefungen im oberen Einlegebereich erhalten. Im Fixierungsbereich verlaufen die sich gegenüberliegenden Öffnungskontur der Wandungen der Vertiefungen parallel zueinander.

In einer besonders bevorzugten Ausgestaltung der Erfindung erstreckt sich der Fixierungsbereich über eine Höhe von 0,1 mm bis 1 mm, insbesondere von 0,2 mm bis 0,4 mm. Die Höhe des Fixierungsbereichs ist in den vorgenannten Bereichen ausreichend, um die vorgenannte Funktion des Fixierungsbereichs zu erfüllen und gleichzeitig bleibt der obere Einlegebereich ebenfalls ausreichend dimensioniert, um dessen vorgenannte Funktion zu erfüllen.

In einer besonders bevorzugten Ausgestaltung der Erfindung umfasst die Probenvorrichtung Probeneinsätze mit einem hohlzylinderförmigen, einseitig eine obere Gehäuseöffnung aufweisenden Gehäuse, einem der Gehäuseöffnung gegenüberliegenden, membranartigen Gehäuseboden, sowie am Gehäuse angeordneten, nach außen abstehenden, zueinander beanstandeten Tragarmen, wobei die Breite der Tragarme um 0,1 mm bis 0,5 mm, insbesondere 0,25 mm bis 0,35 mm geringer ist, als die Breite der Vertiefung auf der Höhe, auf der die Tragarme in den Vertiefungen eingelegt sind. Die Tragarmen haben somit seitlich nur ein sehr geringes Spiel in den Vertiefungen, sodass die durch das Einsetzen des Probeneinsatzes in den Probenraum des Einzelwells gewählte Positionierung kaum noch durch äußere Einflüsse geändert wird. Messungen, bei denen die Lageunveränderlichkeit des im Probeneinsatz befindlichen Zellmaterial relativ zur Multiwell-Platte von Bedeutung ist, wie beispielsweise TEER-Messungen, sind dadurch weniger fehleranfällig.

Mit Vorteil haben die Vertiefungen eine Tiefe von 1,5 mm bis 3 mm, insbesondere 1,7 mm bis 2,3 mm und besonders bevorzugt von 1,9 mm bis 2,1 mm. Dieser Bereich der Tiefe hat sich als besonders vorteilhaft für das Erreichen der eingangs genannten Vorteile der Vertiefungen herausgestellt.

Die Multiwell-Platte weist bevorzugt 6, 12 oder 24 Einzelwells auf, die nebeneinander in gleichmäßigen Reihen positioniert sind.

Weitere Einzelheiten und Vorteile der Erfindung sind den Unteransprüchen sowie dem nachfolgend beschriebenen Ausführungsbeispiel zu entnehmen. Es zeigen:
- Fig. 1: eine erfindungsgemäße Probenvorrichtung, mit einer Multiwell-Platte mit eingesetzten Probeneinsätzen und mit einem Deckel,
- Fig. 2: die Multiwell-Platte aus Fig. 1 in einer Draufsicht,
- Fig. 3: den Gegenstand aus Fig. 2 in einer geschnittenen Darstellung nach der Linie III-III,
- Fig. 4: eine Detailvergrößerung des Gegenstands aus Fig. 2 aus einer Perspektive aus Richtung des Pfeils IV in Fig. 2,
- Fig. 5: den Deckel aus Fig. 1 die Innenseite des Deckels zeigend und
- Fig. 6: den Gegenstand aus Fig. 5 in einer Schnittdarstellung nach der Linie VI-VI.

Die nachfolgend dargestellten Merkmale der Probenvorrichtung können auch in anderen Kombinationen Gegenstand der Erfindung sein. Gleichwirkende Elemente sind im Folgenden mit einer einheitlichen Bezugsziffer versehen, sofern dies sinnvoll ist.

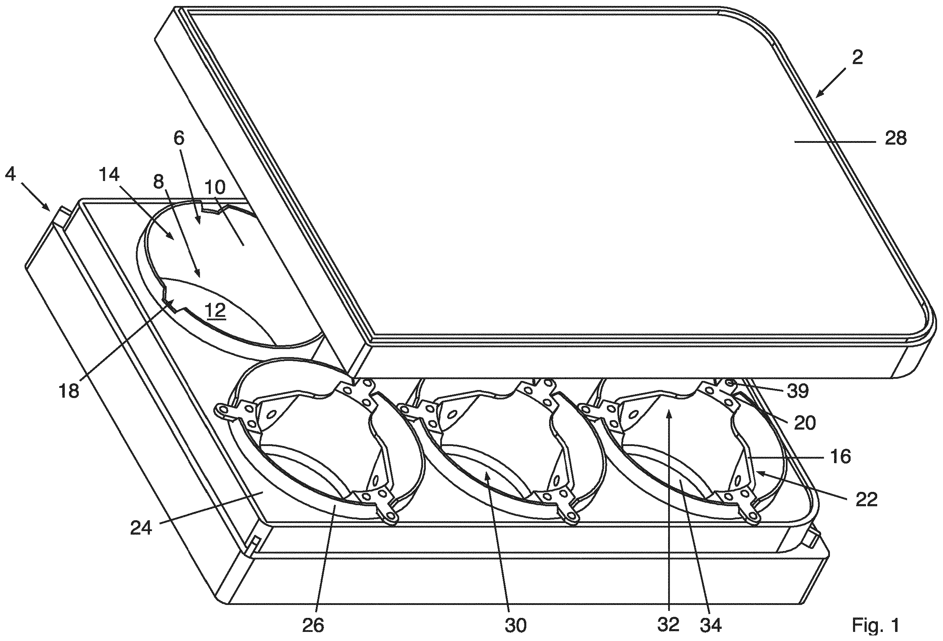

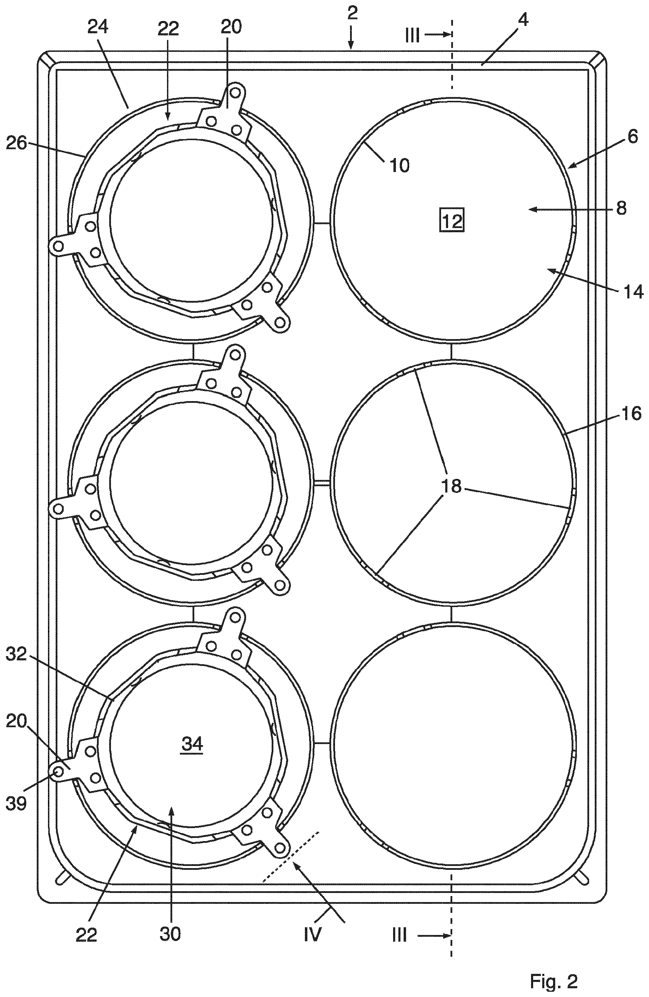

Fig.1 zeigt eine erfindungsgemäße Probenvorrichtung 2 mit einer Multiwell-Platte 4, die eine Mehrzahl fest mit der Multiwell-Platte 4 verbundener Einzelwells 6 aufweist. Die Multiwell-Platte 4 ist in Fig. 2 in einer Draufsicht dargestellt. Jedes Einzelwell 6 weist einen gegenüber den weiteren Einzelwells 6 abgegrenzten Probenraum 8 für eine Nährlösung auf, der umseitig von einer Wandung 10 und unten von einem sich an die Wandung 10 anschließenden Boden 12 begrenzt ist und der über eine dem Boden 12 gegenüberliegende Öffnung 14 zugänglich ist. Die Öffnung 14 ist von einer die Wandung 10 begrenzenden Öffnungskontur 16 umfasst. Jede Öffnungskontur 16 der Wandung 10 eines Einzelwells 6 weist diesem zugeordnete Vertiefungen 18 zum Einlegen von Tragarmen 20 eines Probeneinsatzes 22 auf und ist frei von benachbarten Einzelwells 6 zugeordneten Vertiefungen 18. In Fig. 1 und Fig. 2 sind drei der insgesamt sechs Einzelwells 6 mit Probeneinsätzen 22 besetzt.

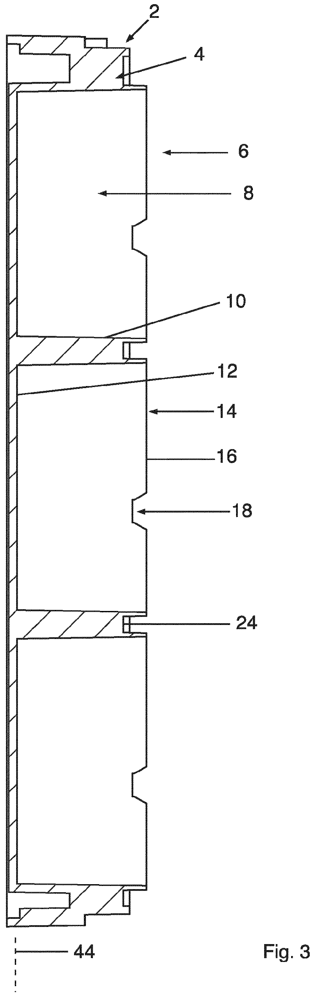

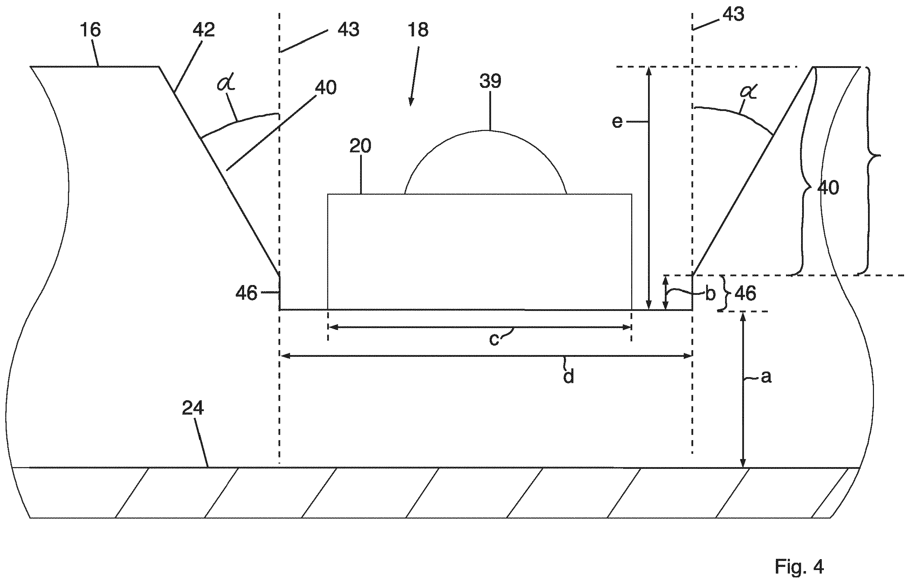

Jede der Wandungen 10 weist einen gegenüber dem an das jeweilige Einzelwell 6 unmittelbar angrenzenden Umfassungsbereich 24 erhöhten Sicherheitsrand 26 auf. Der Sicherheitsrand 26 ist in der Schnittdarstellung der Multiwell-Platte 4 in Fig. 3 und noch besser in der teilweise geschnittenen Detailansicht in Fig. 4 gut zu erkennen. Der Sicherheitsrand 26 jedes Einzelwells 6 ist um wenigstens 0,5 mm, insbesondere um wenigstens 1 mm, gegenüber dem an das jeweilige Einzelwell 6 unmittelbar angrenzenden Umfassungsbereich 24 erhöht. Der Umfassungsbereich 24 ist in Fig. 4 geschnitten dargestellt. Dort ist die vorgenannte Mindesthöhe a des Sicherheitsrands 26 von wenigstens 0,5 mm und insbesondere von wenigstens 1 mm veranschaulicht. Dieser Wert wird auch im Bereich der Vertiefungen 18 nicht unterschritten.

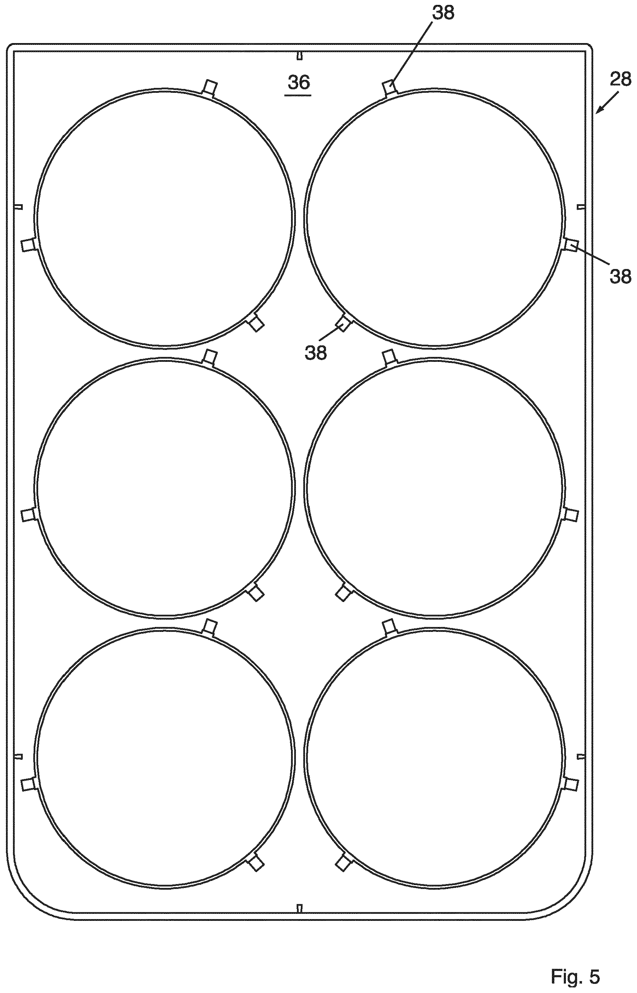

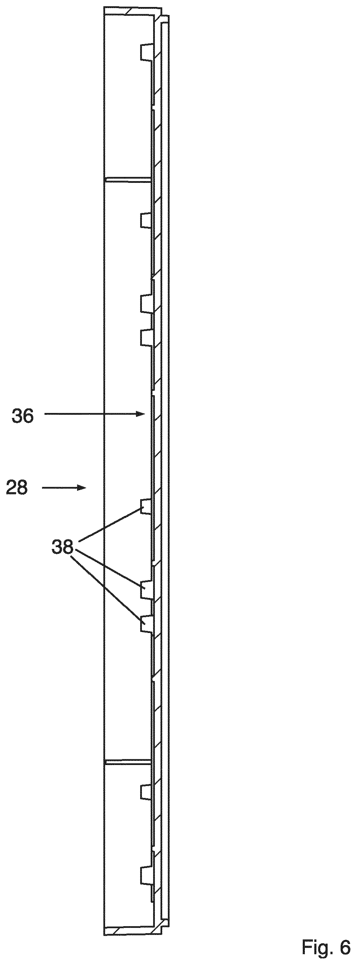

Die Probenvorrichtung 2 in Fig.1 umfasst zudem einen an die Form der Multiwell-Platte 4 angepassten Deckel 28 und Probeneinsätze 22 mit einem hohl zylinderförmigen, einseitig eine obere Gehäuseöffnung 30 aufweisenden Gehäuse 32, einem der Gehäuseöffnung 30 gegenüberliegenden, membranartigen Gehäuseboden 34, sowie am Gehäuse 32 angeordneten, nach außen abstehenden, zueinander beabstandeten Tragarmen 20. Der Deckel 28 ist in Fig. 5 in einer Unteransicht auf die Innenseite 36 des Deckels 28 dargestellt. Fig. 6 zeigt eine Schnittdarstellung durch den Deckel 28 nach der Linie VI-VI. Der Deckel 28 weist innenseitig Niederhalter 38 auf, die im geschlossenen Zustand der Probenvorrichtung 2 mit auf der Multiwell-Platte 4 aufgesetztem Deckel 28 jeweils oberhalb eines Tragarmes 20 positioniert sind. Bevorzugt weisen die Tragarme 20 auf der dem Gehäuseboden 34 abgewandten Oberfläche zumindest einen Vorsprung 39 auf und der Niederhalter 38 ist jeweils über diesem Vorsprung 39 positioniert, wenn der Deckel 28 auf der Multiwell-Platte 4 aufgesetzt ist.

Der Abstand zwischen den Niederhaltern 38 und den Tragarmen 20 beträgt im geschlossenen Zustand der Probenvorrichtung 2 mit auf der Multiwell-Platte 4 aufgesetztem Deckel 28 nicht mehr als 0,3 mm. Besonders bevorzugt liegen die Niederhalter 38 in diesem Zustand auf den Tragarmen 20 auf.

In Fig. 4 ist der Bereich einer Vertiefung 18 in einer Detailansicht dargestellt. Jede der Vertiefungen 18 in der Öffnungskontur 16 hat im oberen Bereich einen Einlegebereich 40, der zwei sich gegenüberliegende Schenkel 42 aufweist, die konisch zueinander verlaufen. Die Schenkel 42 schließen jeweils einen Winkel α mit einer Senkrechten 43 zur Bodenebene 44, in der die Böden 12 der Einzelwells 6 liegen, von 15° bis 45°, insbesondere von 25° bis 35° ein.

Die Öffnungskontur 16 weist im unteren Bereich der Vertiefungen 18 jeweils einen Fixierungsbereich 46 auf, in dem die Öffnungskontur 16 beiderseits jeweils senkrecht zur Bodenebene 44 verläuft. Der Fixierungsbereich 46 erstreckt sich über eine Höhe b von 0,1 mm bis 1 mm, insbesondere von 0,2 mm bis 0,4 mm.

In Fig. 4 ist auch ein Tragarm 20, eingelegt in die Vertiefung 18 dargestellt. Die Breite c der Tragarme 20 ist um 0,1 mm bis 0,5 mm, insbesondere 0,25 mm bis 0,35 mm geringer als die Breite d der Vertiefung 18 auf der Höhe, auf der die Tragarme 20 in den Vertiefungen 18 eingelegt sind.

Die Vertiefungen 18 haben eine Tiefe e von 1,5 mm bis 3 mm, insbesondere 1,7 mm bis 2,3 mm, wie in Fig. 4 veranschaulicht.

### Bezugsziffern

- 2: Probenvorrichtung
- 4: Multiwell-Platte
- 6: Einzelwell
- 8: Probenraum
- 10: Wandung
- 12: Boden
- 14: Öffnung
- 16: Öffnungskontur
- 18: Vertiefung
- 20: Tragarm
- 22: Probeneinsatz
- 24: Umfassungsbereich
- 26: Sicherheitsrand
- 28: Deckel
- 30: Gehäuseöffnung
- 32: Gehäuse
- 34: Gehäuseboden
- 36: Innenseite des Deckels
- 38: Niederhalter
- 39: Vorsprung am Tragarm
- 40: Einlegebereich
- 42: Schenkel
- α: Winkel zwischen Schenkel und Senkrechte zur Bodenebene
- 43: Senkrechte zur Bodenebene
- 44: Bodenebene
- 46: Fixierungsbereich
- a: Mindesthöhe des Sicherheitsrands
- b: Höhe des Fixierungsbereichs
- c: Breite der Tragarme
- d: Breite der Vertiefung
- e: Tiefe der Vertiefungen

## Patentansprüche

1. Probenvorrichtung (2) mit einer Multiwell-Platte (4), die eine Mehrzahl fest mit der Multiwell-Platte (4) verbundener Einzelwells (6) aufweist, wobei jedes Einzelwell (6) einen gegenüber den weiteren Einzelwells (6) abgegrenzten Probenraum (8) für eine Nährlösung aufweist, der umseitig von einer Wandung (10) und unten von einem sich an die Wandung (10) anschließenden Boden (12) begrenzt ist und der über eine dem Boden (12) gegenüberliegende Öffnung (14) zugänglich ist, die von einer die Wandung (10) begrenzenden Öffnungskontur (16) umfasst ist, und wobei jede Öffnungskontur (16) der Wandung (10) eines Einzelwells (6) diesem zugeordnete Vertiefungen (18) zum Einlegen von Tragarmen (20) eines Probeneinsatzes (22) aufweist und frei von benachbarten Einzelwells (6) zugeordneten Vertiefungen (18) ist, und wobei die Probenvorrichtung (2) einen an die Form der Multiwell-Platte (4) angepassten Deckel (28) und Probeneinsätze mit einem hohlzylinderförmigen, einseitig eine obere Gehäuseöffnung (30) aufweisenden Gehäuse (32), einem der Gehäuseöffnung (30) gegenüberliegenden, membranartigen Gehäuseboden (34), sowie am Gehäuse (32) angeordneten, nach außen abstehenden, zueinander beabstandeten Tragarmen (20) umfasst, wobei der Deckel (28) innenseitig Niederhalter (38) aufweist, die im geschlossenen Zustand der Probenvorrichtung (2) mit auf der Multiwell-Platte (4) aufgesetztem Deckel (28) oberhalb jedes Tragarmes (20) positioniert sind.

2. Probenvorrichtung (2) nach Anspruch 1, **dadurch gekennzeichnet, dass** jede der Wandungen (10) einen gegenüber dem an das jeweilige Einzelwell (6) unmittelbar angrenzenden Umfassungsbereich (24) erhöhten Sicherheitsrand (26) aufweist.

3. Probenvorrichtung (2) nach Anspruch 2, **dadurch gekennzeichnet, dass** der Sicherheitsrand (26) jedes Einzelwells (6) um wenigstens 0,5 mm gegenüber dem an das jeweilige Einzelwell (6) unmittelbar angrenzenden Umfassungsbereich (24) erhöht ist.

4. Probenvorrichtung (2) nach Anspruch 3, **dadurch gekennzeichnet, dass** der Abstand zwischen den Niederhaltern (28) und den Tragarmen (20) im geschlossenen Zustand der Probenvorrichtung (2) mit auf der Multiwell-Platte (4) aufgesetztem Deckel nicht mehr als 0,3 mm beträgt.

5. Probenvorrichtung (2) nach einem der Ansprüche 3 oder 4, **dadurch gekennzeichnet, dass** die Niederhalter (28) im geschlossenen Zustand der Probenvorrichtung (2) mit auf der Multiwell-Platte (4) aufgesetztem Deckel (28) auf den Tragarmen (20) aufliegen.

6. Probenvorrichtung (2) nach einem der vorangehenden Ansprüche, **dadurch gekennzeichnet, dass** jede der Vertiefungen (18) in der Öffnungskontur (16) im oberen Bereich einen Einlegebereich (40) hat, der zwei sich gegenüberliegende Schenkel (42) aufweist, die konisch zueinander verlaufen und jeweils einen Winkel (α) zu einer Senkrechten (43) zur Bodenebene (44), in der die Böden (12) der Einzelwells (6) liegen, von 15° bis 45°, insbesondere von 25° bis 35° einschließen.

7. Probenvorrichtung (2) nach Anspruch 6, **dadurch gekennzeichnet, dass** die Öffnungskontur (16) im unteren Bereich der Vertiefungen (18) jeweils einen Fixierungsbereich (46) aufweist, in dem die Öffnungskontur (16) beiderseits jeweils senkrecht zur Bodenebene (44) verläuft.

8. Probenvorrichtung (2) nach Anspruch 7, **dadurch gekennzeichnet, dass** sich der Fixierungsbereich (46) über eine Höhe (b) von 0,1 mm bis 1 mm, insbesondere von 0,2 mm bis 0,4 mm erstreckt.

9. Probenvorrichtung (2) nach einem der vorangehenden Ansprüche, **dadurch gekennzeichnet, dass** die Probenvorrichtung (2) Probeneinsätze (22) mit einem hohlzylinderförmigen, einseitig eine obere Gehäuseöffnung (30) aufweisenden Gehäuse (32), einem der Gehäuseöffnung (30) gegenüberliegenden, membranartigen Boden (34), sowie am Gehäuse (32) angeordneten, nach außen abstehenden, zueinander beanstandeten Tragarmen (20) umfasst, wobei die Breite (c) der Tragarme um 0,1 mm bis 0,5 mm, insbesondere 0,25 mm bis 0,35 mm geringer ist, als die Breite (d) der Vertiefung (18) auf der Höhe, auf der die Tragarme (20) in den Vertiefungen (18) eingelegt sind.

10. Probenvorrichtung (2) nach einem der vorangehenden Ansprüche, **dadurch gekennzeichnet, dass** die Vertiefungen (18) eine Tiefe (e) von 1,5 mm bis 3 mm, insbesondere 1,7 mm bis 2,3 mm haben.

## Claims

1. Sampling device (2) having a multi-well plate (4) which has a plurality of individual wells (6) which are connected fixedly to the multi-well plate (4), wherein each individual well (6) has a sample space (8) for a nutrient solution that is delimited in relation to the further individual wells (6), said sample space being delimited peripherally by a wall (10) and at the bottom by a base (12) which adjoins the wall (10) and being accessible via an opening (14) which is situated opposite the base (12) and which is surrounded by an opening contour (16) which delimits the wall (10), and wherein each opening contour (16) of the wall (10) of an individual well (6) has depressions (18) assigned thereto for placement of supporting arms (20) of a sample insert (22) and is free of depressions (18) assigned to neighbouring individual wells (6), and wherein the sampling device (2) comprises a cover (28), which is adapted to the shape of the multi-well plate (4), and sample inserts, which have a hollow-cylindrical housing (32) having an upper housing opening (30) at one side, have a membrane-like housing base (34) situated opposite the housing opening (30) and have outwardly projecting, mutually spaced-apart supporting arms (20) arranged on the housing (32), wherein the cover (28) has on the inside holding-down means (38) which, in the closed state of the sampling device (2) with the cover (28) fitted on the multi-well plate (4), are positioned above each supporting arm (20).

2. Sampling device (2) according to Claim 1, **characterized in that** each of the walls (10) has a safety edge (26) which, in relation to the surrounding region (24) that is directly adjacent to the respective individual well (6), is raised.

3. Sampling device (2) according to Claim 2, **characterized in that** the safety edge (26) of each individual well (6) is raised by at least 0.5 mm in relation to the surrounding region (24) directly adjacent to the respective individual well (6).

4. Sampling device (2) according to Claim 3, **characterized in that**, in the closed state of the sampling device (2) with the cover fitted on the multi-well plate (4), the distance between the holding-down means (28) and the supporting arms (20) is not greater than 0.3 mm.

5. Sampling device (2) according to either of Claims 3 and 4, **characterized in that**, in the closed state of the sampling device (2) with the cover (28) fitted on the multi-well plate (4), the holding-down means (28) bear on the supporting arms (20).

6. Sampling device (2) according to one of the preceding claims, **characterized in that** each of the depressions (18) in the opening contour (16) has in the upper region a placement region (40) having two oppositely situated legs (42) which extend conically in relation to one another and which each include an angle (α) to a normal (43) to the base plane (44), which contains the bases (12) of the individual wells (6), of 15° to 45°, in particular of 25° to 35°.

7. Sampling device (2) according to Claim 6, **characterized in that** the opening contour (16) has in the lower region of the depressions (18) in each case one fixing region (46) in which the opening contour (16) extends perpendicularly to the base plane (44) on each side.

8. Sampling device (2) according to Claim 7, **characterized in that** the fixing region (46) extends over a height (b) of 0.1 mm to 1 mm, in particular of 0.2 mm to 0.4 mm.

9. Sampling device (2) according to one of the preceding claims, **characterized in that** the sampling device (2) comprises sample inserts (22) which have a hollow-cylindrical housing (32) having an upper housing opening (30) at one side, have a membrane-like base (34) situated opposite the housing opening (30) and have outwardly projecting, mutually spaced-apart supporting arms (20) arranged on the housing (32), where the width (c) of the supporting arms is 0.1 mm to 0.5 mm, in particular 0.25 mm to 0.35 mm, smaller than the width (d) of the depression (18) at the height at which the supporting arms (20) are placed in the depressions (18).

10. Sampling device (2) according to one of the preceding claims, **characterized in that** the depressions (18) have a depth (e) of 1.5 mm to 3 mm, in particular 1.7 mm to 2.3 mm.

## Revendications

1. Dispositif d'échantillonnage (2), comprenant une plaque de microtitrage (4) qui présente une pluralité de puits individuels (6) reliés solidement à la plaque de microtitrage (4), dans lequel chaque puits individuel (6) présente un espace d'échantillonnage (8) pour une solution nutritive, délimité par rapport aux autres puits individuels (6), qui est délimité en périphérie par une paroi (10) et en bas par un fond (12) adjacent à la paroi (10), et qui est accessible par une ouverture (14) opposée au fond (12) qui est entourée par un contour d'ouverture (16) délimitant la paroi (10), et dans lequel chaque contour d'ouverture (16) de la paroi (10) d'un puits individuel (6) présente des creux (18) qui lui sont associés pour insérer des bras porteurs (20) d'un insert d'échantillon (22) et est exempt de creux (18) associés à des puits individuels (6) voisins, et dans lequel le dispositif d'échantillonnage (2) comprend un couvercle (28) adapté à la forme de la plaque de microtitrage (4) et des inserts d'échantillons pourvus d'un boîtier (32) cylindrique creux, présentant d'un côté une ouverture de boîtier supérieure (30), un fond de boîtier (34) de type membrane, opposé à l'ouverture de boîtier (30), ainsi que des bras porteurs (20) disposés sur le boîtier (32), s'écartant vers l'extérieur, mutuellement espacés, dans lequel le couvercle (28) présente sur la face intérieure des dispositifs de retenue (38) qui sont positionnés, à l'état fermé du dispositif d'échantillonnage (2), le couvercle (28) étant posé sur la plaque de microtitrage (4), au-dessus de chaque bras porteur (20).

2. Dispositif d'échantillonnage (2) selon la revendication 1, **caractérisé en ce que** chacune des parois (10) présente un bord de sécurité (26) surélevé par rapport à la zone périphérique (24) directement adjacente au puits individuel (6) respectif.

3. Dispositif d'échantillonnage (2) selon la revendication 2, **caractérisé en ce que** le bord de sécurité (26) de chaque puits individuel (6) est surélevé d'au moins 0,5 mm par rapport à la zone périphérique (24) directement adjacente au puits individuel (6) respectif.

4. Dispositif d'échantillonnage (2) selon la revendication 3, **caractérisé en ce que** la distance entre les dispositifs de retenue (28) et les bras porteurs (20) ne mesure pas plus de 0,3 mm à l'état fermé du dispositif d'échantillonnage (2), le couvercle étant posé sur la plaque de microtitrage (4).

5. Dispositif d'échantillonnage (2) selon l'une quelconque des revendications 3 ou 4, **caractérisé en ce qu'**à l'état fermé du dispositif d'échantillonnage (2), le couvercle (28) étant posé sur la plaque de microtitrage (4), les dispositifs de retenue (28) reposent sur les bras porteurs (20).

6. Dispositif d'échantillonnage (2) selon l'une quelconque des revendications précédentes, **caractérisé en ce que** chacun des creux (18) présente dans le contour d'ouverture (16) dans la partie supérieure une zone d'insertion (40) qui présente deux branches (42) opposées qui s'étendent de manière conique l'une par rapport à l'autre et forment respectivement un angle (α) de 15° à 45°, en particulier de 25° à 35°, par rapport à une verticale (43) au plan de fond (44) dans lequel se trouvent les fonds (12) des puits individuels (6).

7. Dispositif d'échantillonnage (2) selon la revendication 6, **caractérisé en ce que** le contour d'ouverture (16) présente dans la partie inférieure des creux (18) respectivement une zone de fixation (46) dans laquelle s'étend le contour d'ouverture (16) des deux côtés respectivement perpendiculairement au plan de fond (44).

8. Dispositif d'échantillonnage (2) selon la revendication 7, **caractérisé en ce que** la zone de fixation (46) s'étend sur une hauteur (b) de 0,1 mm à 1 mm, en particulier de 0,2 mm à 0,4 mm.

9. Dispositif d'échantillonnage (2) selon l'une quelconque des revendications précédentes, **caractérisé en ce que** le dispositif d'échantillonnage (2) comprend des inserts d'échantillon (22) pourvus d'un boîtier (32) cylindrique creux, présentant d'un côté une ouverture de boîtier supérieure (30), un fond de type membrane (34) opposé à l'ouverture de boîtier (30), ainsi que des bras porteurs (20) disposés sur le boîtier (32) s'écartant vers l'extérieur, mutuellement espacés, dans lequel la largeur (c) des bras porteurs est inférieure de 0,1 mm à 0,5 mm, en particulier de 0,25 mm à 0,35 mm à la largeur (d) du creux (18) à la hauteur à laquelle les bras porteurs (20) sont insérés dans les creux (18).

10. Dispositif d'échantillonnage (2) selon l'une quelconque des revendications précédentes, **caractérisé en ce que** les creux (18) ont une profondeur (e) de 1,5 mm à 3 mm, en particulier de 1,7 mm à 2,3 mm.
